# EUROPEAN PATENT SPECIFICATION

(11) **EP 2 684 516 B1**
(45) Date of publication and mention of the grant of the patent: **29.10.2014**
(21) Application number: 12176271.0
(22) Date of filing: 13.07.2012
(51) Int. Cl.: A61B 5/0408, A61B 5/0478

(54) **Improved physiological signal detection device**
Verbesserte physiologische Signalerkennungsvorrichtung
Dispositif amélioré de détection de signal physiologique

(43) Date of publication of application: 15.01.2014
(73) Proprietor: King's Metal Fiber Technologies Co., Ltd., Taichung City (TW)
(72) Inventor: Huang, Hong-Hsu, Taipei City (TW); Su, I-Chen, Taipei City (TW); Yang, Shun-Tung, Taipei City (TW)
(74) Representative: Viering, Jentschura & Partner Patent- und Rechtsanwälte

(56) References cited:
- EP-A1- 2 407 096
- DE-A1-102009 017 179
- US-A1- 2003 176 908

## Description

### FIELD OF THE INVENTION

The present invention relates to an improved physiological signal detection device, and in particular to a physiological signal detection device that provides water-absorbing and wet-keeping functions to wet an electrode pad for engagement with a surface of human body in order to facilitate conductive performance for detecting physiological signal and be applicable to detection of physiological signals of human body in a dry area.

### BACKGROUND OF THE INVENTION

Nowadays, to detect physiological signals of human body, such as heart beat and brain wave, a plurality of electrode pads of physiological detection equipment is attached to (or worn on) various sites on a surface of human body (also referred to as body surface). These electrode pads detect the current that spreads to the peripheral tissues or body surface occurring when nervous impulses (namely variation of membrane potential) passes through human body organs (such as heart and head). The current is then transmitted by electrical wires to the physiological detection equipment to be converted into data to be displayed. In this way, the condition of an inspected portion (such as heart rate and variation of brain wave) can be realized.

Examples of electrode pads for physiological signal detection are disclosed in EP-A-2 407 096, US-A-2003/176908 or DE-A-10 2009 017179. These electrode pads have a base layer with at least one electrode pad on a top surface and a water absorption unit positioned between the electrode pad and the base layer.

The conventional electrode pad has a structure comprising a base layer (such as a layer of conductive adhesive) and an electrically conductive portion bonded to the base layer. To use in inspecting physiological signals, the body surface (such as skin) is made wet by the stickiness of the base layer or through additional application of a layer of aqueous gel that is electrically conductive in order to help the current on the body surface to flow through the base layer, the electrically conductive portion, and the electrical wires to the physiological detection equipment. On the other hand, to carry out electrotherapy, electrical current is transmitted from the physiological detection equipment to the body surface to penetrate into the body surface to simulate the portion to be treated.

However, the base layer of the conventional electrode pad, as well as the aqueous gel used in combination therewith, is generally not air permeable and may often cause allergy, and thus resulting in uncomfortableness of use. Further, skin chips (for example in condition of dry skin) and grease (in condition of oily skin) are often generated on the human body surface and may easily get stuck to the base layer and interferes with conduction of electrical current. Further, human body has body temperature, which may often causes loss (evaporation) of body surface humidity, or the gel used may get dried and is no longer capable of a humid condition, leading to separation of the adhesive from the base layer. This also interferes with the conduction of electrical current and makes it hard to detect physiological signals. Particularly in a dry condition, the humidity is even harder to keep and interruption of detection results. It may also cause cracking of the base layer.

Further, when multiple conventional electrode pads are used, if these conventional electrode pads are placed too close to each other, then they may get contact with each other and short-circuit may result.

In view of these problems, the present invention aims to provide an improved physiological signal detection device that comprises water-absorbing and wet-keeping electrical pads and is set in engagement with surface of human body to improve electrical conduction for physiological signal detection and to facilitate use in a dry area to detect physiological signals of an inspection subject and to increase convenience of use.

### SUMMARY OF THE INVENTION

An object of the present invention is to provide an improved physiological signal detection device that has water-absorbing and wet-keeping functions to prevent fast loss of water and help wetting an electrode pad to thereby improve electrical conduction for physiological signal detection and facilitate use in a dry area to detect physiological signals of an inspection subject and to increase convenience of use.

Another object of the present invention is to provide an improved physiological signal detection device that comprises an edge enclosure to provide the functions of positioning and improving comfortableness and aesthetics and also reduce the influence thereof to conduction of electrical current and also reduce noise interference.

A further object of the present invention is to provide an improved physiological signal detection device that forms distinct projections after absorbing water so as to be easy to attach to and securely bond to human body surface.

To realize the above objects, the present invention provides a physiological signal detection device that comprises a base layer, at least one electrode pad, which is positioned on a top surface of the base layer, he electrode pad and the base layer forming a first receiving compartment therebetween, and at least one water absorption unit, which is positioned in the first receiving compartment. The water absorption unit has a top engaging the electrode pad, and the water absorption unit has a bottom engaging the base layer. As such, water absorbing and wet keeping functions are achieved, by which fast loss of water is prevented and wetting of the electrode pad is enhanced. Further, the water absorption unit is capable of bulging by absorbing water to raise the electrode pad, so that the electrode pad is capable of easy contact and tight engagement with human body surface to ease the detection of physiological signal of an inspection subject and improve convenience of use. Further, an edge enclosure band is also included to provide a function of positioning, improving comfortableness and aesthetics, and reducing influence on conduction of electrical current and lowering noise interference.

### BRIEF DESCRIPTION OF THE DRAWINGS

The present invention will be apparent to those skilled in the art by reading the following description of preferred embodiments thereof with reference to the drawings, in which:
Figure 1 is a perspective view showing a physiological signal detection device according to the present invention;
Figure 2 is a cross-sectional view taken along line A-A of Figure 1;
Figure 3 is a cross-sectional view, in an enlarged form, taken along line B-B of Figure 1;
Figure 4 is a schematic view showing each electrode pad of the physiological signal detection device shown in Figure 2 is provided with extension conductor and a base layer being formed of an upper water-penetrating layer and a lower water-proof layer overlapping each other;
Figure 5 is an exploded view showing the physiological signal detection device of Figure 1 further comprising an edge enclosure band, connection band being provided between electrode pads, each electrode pad being provided with an extension conductor
Figure 6 is a schematic view showing the physiological signal detection device shown in Figure 2 further comprising an edge enclosure band;
Figure 7 is a schematic view showing a simple structure of the physiological signal detection device according to the present invention;
Figure 8 is an exploded view of the physiological signal detection device shown in Figure 7 further comprising an edge enclosure band;
Figure 9 is a perspective view showing the physiological signal detection device of Figure 8 after being assembled;
Figure 10 is a schematic view showing an embodiment where a water absorption unit of Figure 7 is composed of a water-preservation inner lining in combination with an accommodation sack;
Figure 11 is schematic view showing the accommodation sack of Figure 10 comprises an upper water-penetrating layer and a lower water-proof layer that overlap each other and the water absorption unit absorbing water and getting bulging to show a projecting form; and
Figure 12 is a front view showing the physiological signal detection device according to the present invention coupled to a front-open garment.

### DETAILED DESCRIPTION OF THE PREFERRED EMBODIMENTS

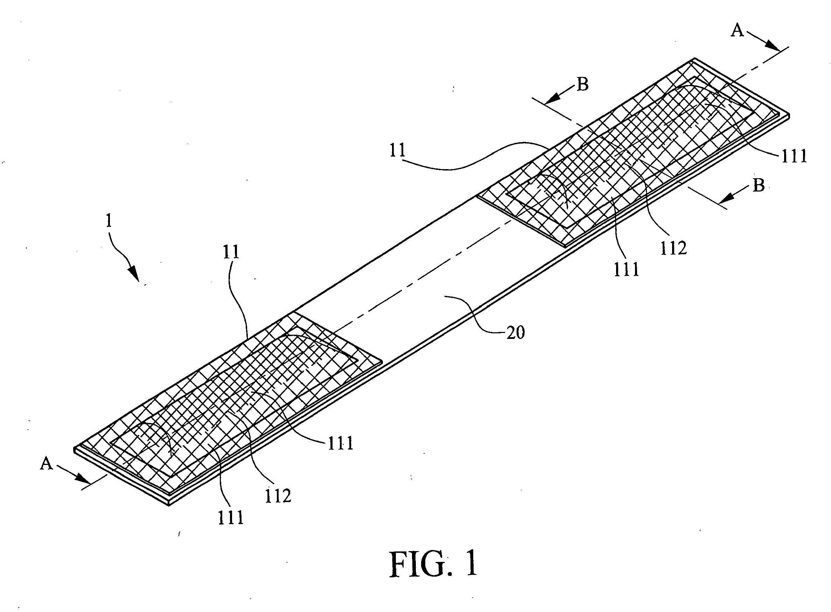

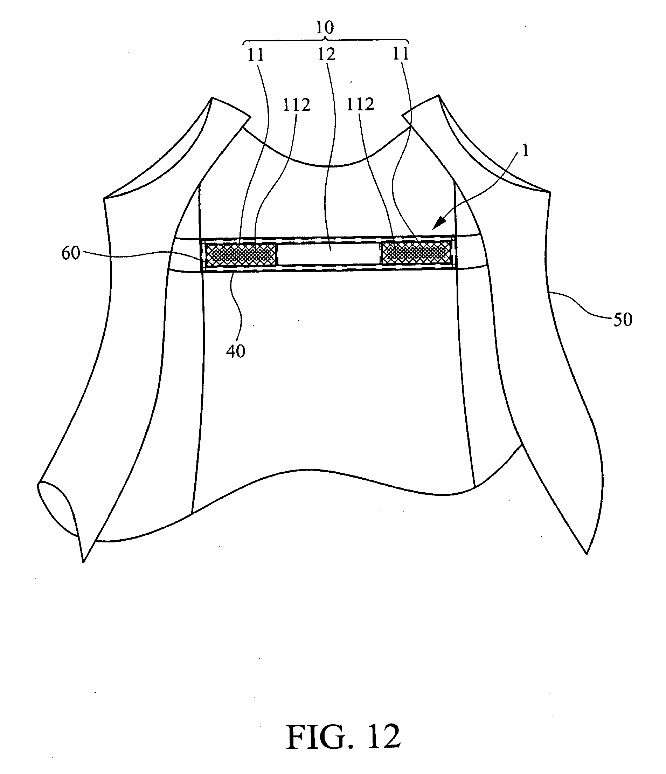

With reference to the drawings and in particular to Figures 1-11, an improved physiological signal detection device 1 according to the present invention comprises a combination of at least one electrode pad 11, a base layer 20, and at least one water absorption unit 30. The electrode pad 11 functions to set in contact with a surface of a portion of human body to be inspected. The water absorption unit 30 is arranged to correspond to the electrode pad 11. The base layer 20 is directly attachable to a wearable article (such as garment 50 of Figure 12).

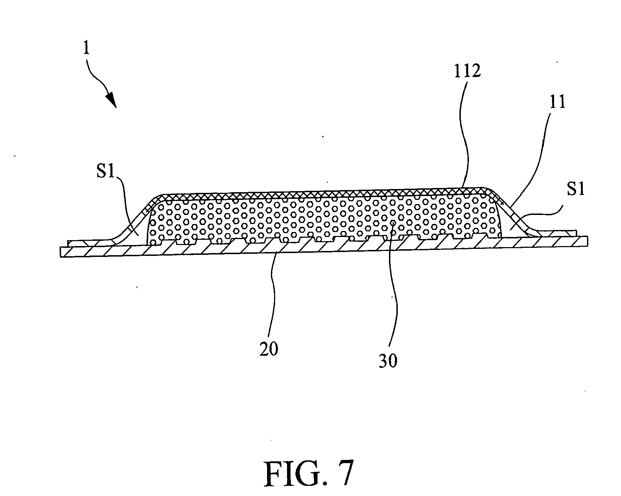

A simple structure of embodiment of the physiological signal detection device 1 according to the present invention is illustrated in Figure 7, which is embodied in the form of a single electrode pad 11. The electrode pad 11 is positioned on a top surface of the base layer 20 in such a way that the electrode pad 11 and the base layer 20 form therebetween a first receiving compartment S1. The water absorption unit 30 is positioned in the first receiving compartment S1. The water absorption unit 30 has a top engaging the electrode pad 11 and a bottom engaging the base layer 20, in order to provide the present invention with a function of water absorption and thus help wetting the electrode pad 11 with the water absorption unit 30.

To actually practice the present invention, the physiological signal detection device 1 according to the present invention can be embodied in various formed according the number of electrode pads 11 used. For an embodiment in which the present invention is embodied with a single electrode pad 11, a wearable article (such as a wrist band or a wrist protector, not shown) to which the physiological signal detection device 1 according to the present invention is combined, is worn on a wrist of a human body, and an electronic device (such as a multimedia playing device, not shown) is also included, with an opposite end of the electronic device being electrically connected to an accessory (such as an earphone, not shown) that is attached to another portion of human body (such as an ear) for grounding (or negative electric pole) purposes so as to form a detection circuit. For an embodiment in which the present invention is embodied with a plurality of electrode pads 11, the physiological signal detection device 1 of the present invention is not limited to being mounted to wrist and may, as shown in Figure 12, be coupled to a garment 50 in such a way that two electrode pads 11 that are mounted inside the garment 50 form a detection circuit.

The electrode pad 11 is formed by weaving a plurality of non-conductive fibrous yarns and a plurality of conductive fibrous yarns. The plurality of non-conductive fibrous yarns and the plurality of conductive fibrous yarns are interwoven to form therebetween a plurality of mesh pores 111. The plurality of conductive fibrous yarns of the electrode pad 11 is woven to form a conductive zone 112 by which contact area is increased. The electrode pad 11 uses the plurality of mesh pores 111 to facilitate penetration of conductive substance (such as water and normal saline) in order to improve conduction of electrical current and also to block foreign objects, such as dusts, from penetrating into the electrode pad 11 for easing subsequent cleaning operation (removing the foreign objects) and maintenance and reducing interference with the conductive zone 112 and minimizing generation of noise and facilitating the water absorption unit 30 absorbing penetrating water to also achieve the function of wet keeping. In the embodiment, the electrode pad 11 is entirely made by weaving a plurality of conductive fibrous yarns to make the entire electrode pad 11 conductive thereby help improving the use for detection.

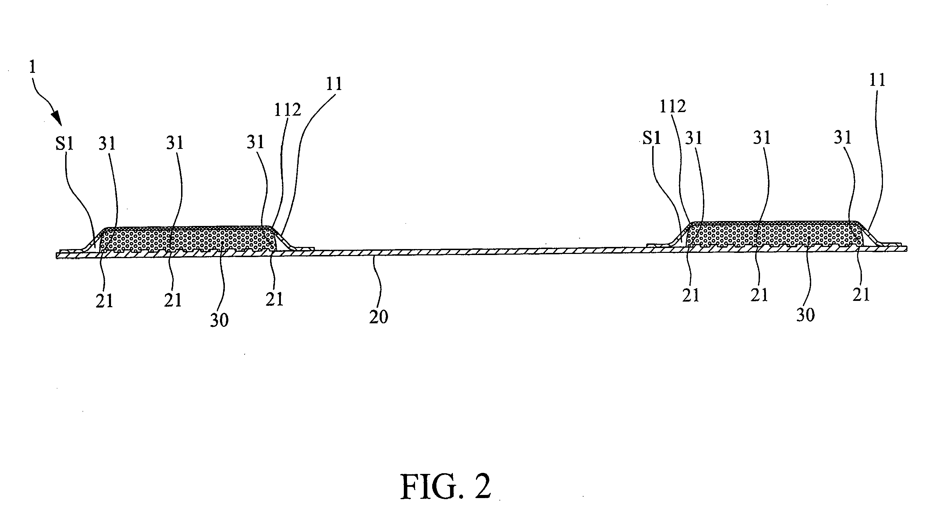

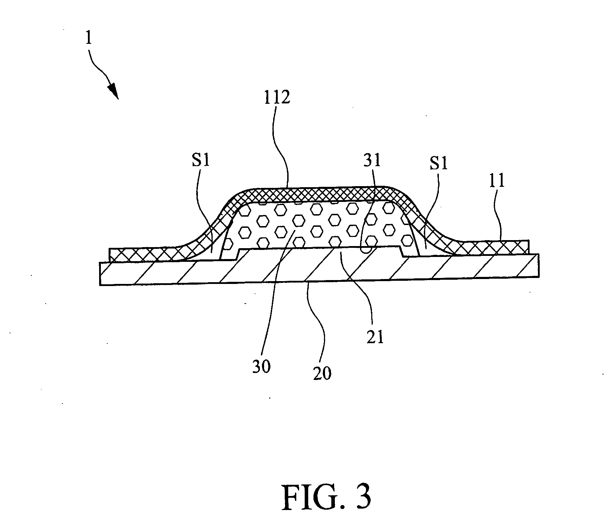

To make the water absorption unit 30 corresponding to and uniformly engaging and thus wetting the electrode pad 11, as shown in Figures 2 and 3, the water absorption unit 30 forms in a bottom thereof a plurality of spaced anti-skidding sections 31 (such as ribs or grooves). The base layer 20 forms, in the top surface thereof, a plurality of spaced counterpart anti-skidding sections 21 (such as grooves or ribs). The counterpart anti-skidding sections 21 respectively correspond to the anti-skidding sections 31. The counterpart anti-skidding sections 21 are respectively engageable with the anti-skidding sections 31 in order to make each water absorption unit 30 stably standing in each first receiving compartment S1 and prevent uneven raised configuration on an outside surface of the electrode pad 11 due to sliding of the water absorption unit 30. In an actual way of practicing the present invention, the counterpart anti-skidding sections 21 can be ribs, while the anti-skidding sections 31 are grooves that are engageable with the ribs.

The water absorption unit 30 is a component made of cotton paper, cotton fabric, silica gel, water-absorbing foam, fluff paste (such as pulp), sodium polyacrylate, or other polymers of propenoic acid that show an equivalent function, or can be embodied as a member of superabsorbent polymers showing an equivalent function. In manufacture, the types used can be increased for easy replacement and avoiding unnecessary constraint to a single type of material.

The water absorption unit 30 is an elastic body having a shape that follows an actual manufacturing process to be a sphere (or a block) for easy manufacturing and to make the water absorption unit 30 of the present invention showing elasticity to be expandable or compressible for easy bending or folding and may resume the original shape thereafter so as to easily comply with the curve of surface of human body when attached to the surface of human body thereby facilitating easy use and replacement.

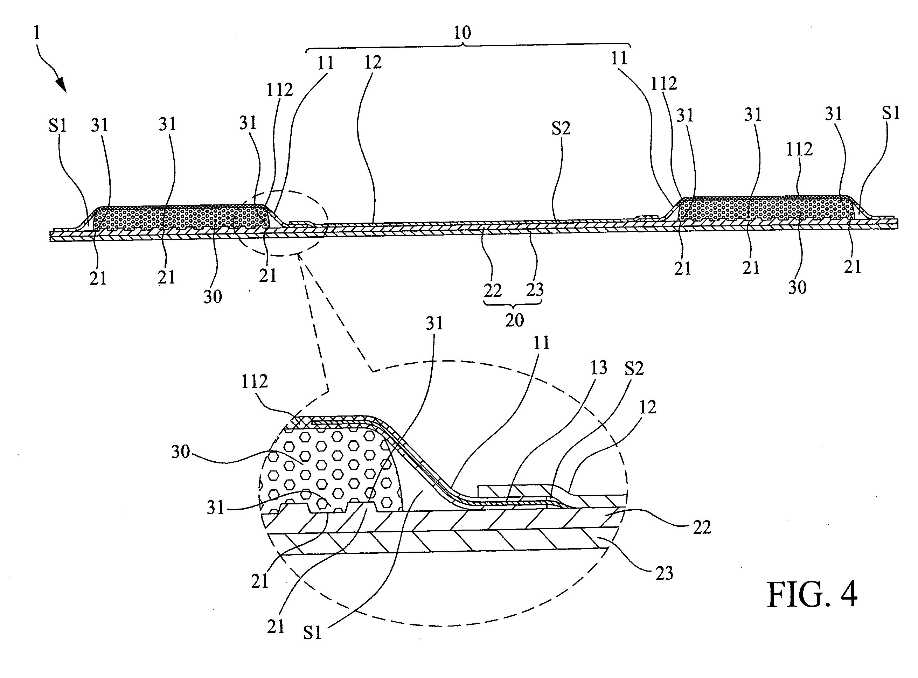

The base layer 20 may comprise a fabric layer or a water-proof layer. In an embodiment where the base layer 20 is made of a fabric layer, the present invention shows air penetrateability for the entirety thereof. On the other hand, the water absorption unit 30 is confined in the first receiving compartment S1 so that water (or humidity), when passing through the electrode pad 11 (or the base layer 20), can be re-absorbed by the water absorption unit 30 so as to effectively prevent water (humidity) from easily flowing out of the physiological signal detection device 1 for continuous wetting the electrode pad 11 for keeping a long period of time of conduction of electrical current. For an embodiment where the base layer 20 is made of a water-proof layer, manufacture can be done with plastic sheet or polyester fiber to provide a water-proof function and prevent loss of water (humidity) through the base layer 20 and also showing a function of wet keeping. However, the base layer 20 of the present invention is not limited to these and as shown in Figure 4, the base layer 20 may comprises an upper water-proof layer 22 and a lower fabric layer 23. The upper water-proof layer 22 (made of polyester fiber) contacts each water absorption unit 30 and provides a water-proof function. The lower fabric layer 23 protects the upper water-proof layer 22. The upper water-proof layer 22 has a top surface that forms the previously discussed counterpart anti-skidding sections 21, while the lower fabric layer 23 is attachable to a garment.

Further, in the instant embodiment, the at least one electrode pad 11 can be a plurality of electrode pads 11, and the at least one water absorption unit 30 can be a plurality of water absorption units 30. The term "a plurality of" refers to a number that is equal to or greater than two. An example of the present invention shown in Figures 2 and 4 comprises two electrode pads 11 used in combination with two water absorption units 30. The two electrode pads 11 are arranged, in a mutually spaced manner, on the top surface of the base layer 20 (see Figure 1) in such a way that each of the electrode pads 11 forms a first receiving compartment S1 with respect to the base layer 20 and the water absorption units 30 are respectively received and retained in the first receiving compartments S1. Each of the water absorption units 30 has a top in engagement with each of the electrode pads 11. Each of the water absorption units 30 has a bottom that is set in engagement with the base layer 20 to facilitate each of the water absorption units 30 to wet each of the electrode pads 11.

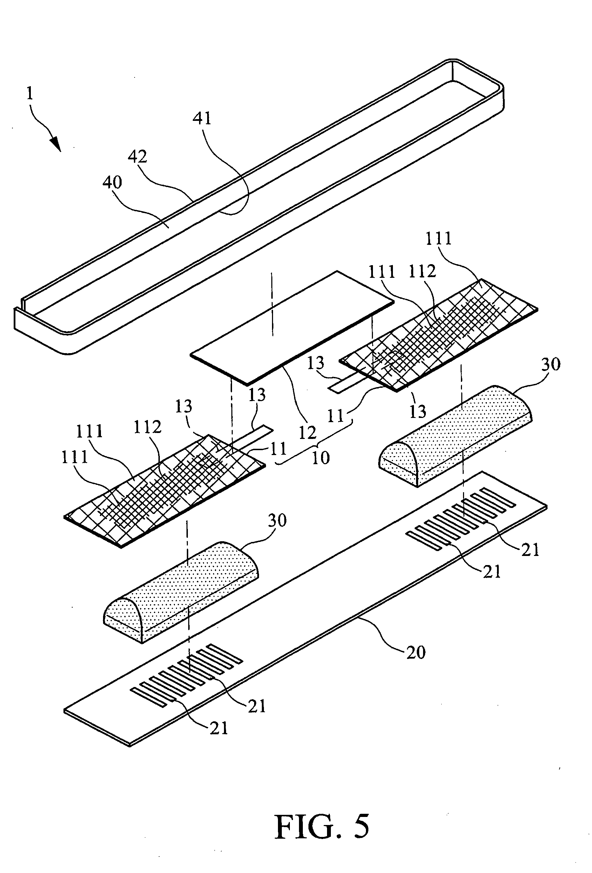

Further, as shown in Figures 4 and 5, at least one connection band 12 is connected between any two adjacent ones of the electrode pads 11, whereby a second receiving compartment S2 is formed between the connection band 12 and the base layer 20. The electrode pads 11 and the connection band 12 are jointed together to form the top layer 10 that corresponds in shape to the base layer 20. In this way, being assisted by the connection band 12, the electrode pads 11 can efficiently attached to opposite sides of a specific portion of human body (such as left and right sides of head, left and right side parts of back, or opposite portions of chest and back of a heart-associated portion) and avoid short-circuit caused by mutual contact occurring between the electrode pads 11.

The second receiving compartment S2 functions to receive and retain other objects (such as electrical wires, sensors, and controllers). In case that no article or object is received in the second receiving compartment S2, the connection band 12 is bonded to the top surface of the base layer 20.

Further, for an electrode pad 11 of a relatively large size, to save expense and to effectively form electrical engagement between the electrode pad 11 and body surface, as shown in Figures 4 and 5, each of the electrode pads 11 is provided with at least one extension conductor 13 (which is particularly shown in the enlarged view at left lower corner of Figure 4) connected thereto. The extension conductor 13 has an end extending into and electrically connected to the electrode pad 11 (namely being electrically connected to the conductive zone 112) and the extension conductor 13 has an opposite end projecting beyond an end of the electrode pad 11, whereby after each water absorption unit 30 absorbs water and bulges, each electrode pad 11 is set in a projecting condition so that easy electrical connection of the extension conductor 13 of the electrode pad 11 can be made with electrical current of the surface of human body. In a practical application, where the present invention is embodied with a single electrode pad 11, the extension conductor 13 discussed above can also be included. Further, when the present invention is embodied with more than two electrode pads 11, based on the sizes thereof, the electrode pads 11 can be selectively provided with and coupled to extension conductors 13.

The extension conductor 13 discussed above for the electrode pad 11 is formed by composing a plurality of conductive yarns that are provided for weaving purposes and are relatively flexible. The extension conductor 13 is electrically connected to an electrical wire (not shown), which has an opposite end electrically connected to physiological detection equipment (not shown), whereby electrical current of body surface flows through the electrode pad 11, the extension conductor 13, and the electrical wire to enter the physiological detection equipment. In a practical application, the extension conductor 13 is not a necessary component and the electrode pad 11 is directly and electrically connected to the electrical wire (not shown).

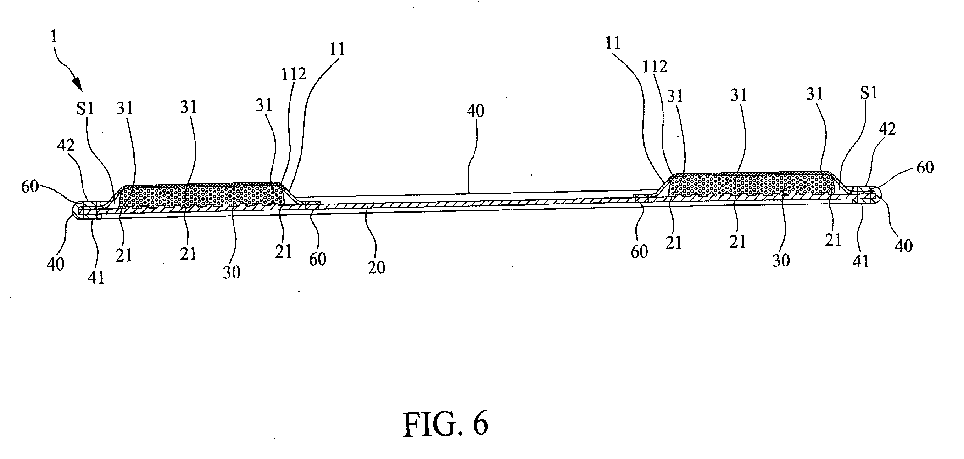

Further, as shown in Figure 5 (in combination with Figure 6), in a preferred embodiment, the present invention further comprises an edge enclosure band 40. The edge enclosure band 40 has a lower flange 41 that is coupled to an edge portion of a bottom surface of the base layer 20 and the edge enclosure band 40 also has an upper flange 42 that is coupled to an edge portion of a top surface of the top layer 10 (which is composed of the connected electrode pads 11 and connection band 12). The edge portion of the top surface of the top layer 10 corresponds to the edge portion of the bottom surface of the base layer 20 so as to allow the edge enclosure band 40 to effect enclosure and retention for improving comfortableness and aesthetics. In the instant embodiment, the connection band 12 is optionally provided based on the practical needs. Thus, the present invention can be embodied as shown in Figure 6 to arrange two mutually-spaced electrode pads 11 on a top surface of the base layer 20 with the upper flange 42 of the edge enclosure band 40 being only coupled to the edge portions of top surfaces of the two electrode pads 11. In this way, when the present invention is attached to a garment 50 (see Figure 12) by sewing, sewn area can be easily identified with the assistance of the edge enclosure band 40, whereby sewing thread 60 is applied along the edge enclosure band 40 to prevent mistakenly penetrating through the water absorption unit and thus damaging the structure. The present invention is applicable in combination with a garment 50, which, when worn by an inspection subject, allows the conductive zones 112 of the two electrode pads 11 to be quickly set corresponding to and conveniently attached, in tight engagement, to the portion to be inspected, eliminating the need for separately attaching the pads.

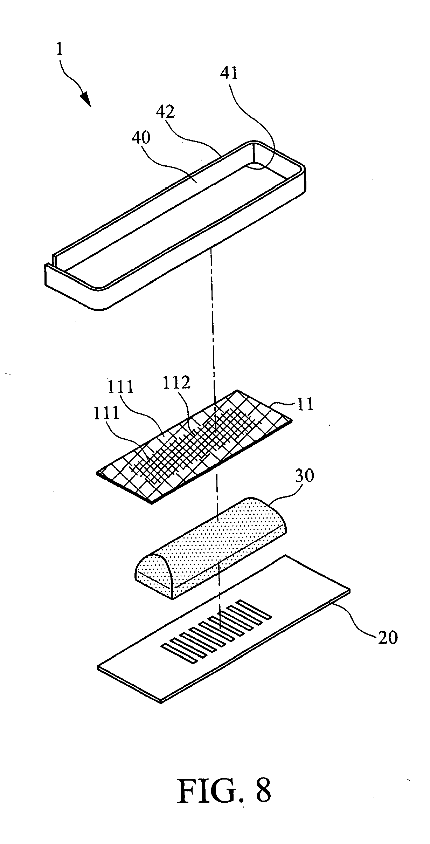

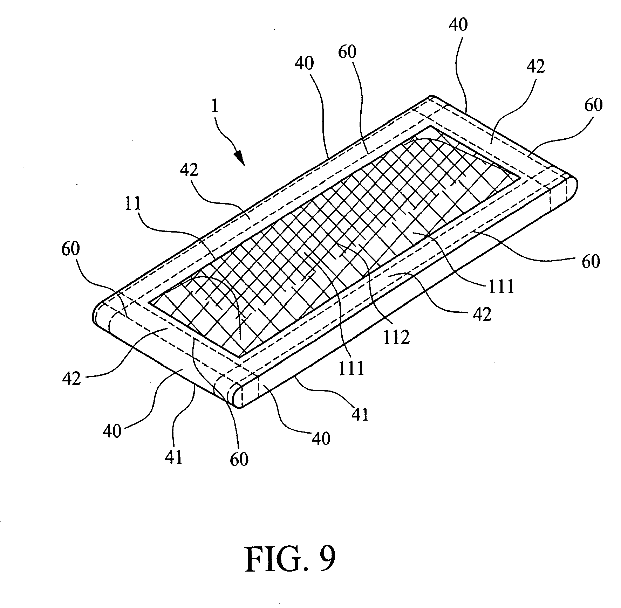

Further, as shown in Figure 8, the edge enclosure band 40 can be embodied in combination with the previously discussed simple structure embodiment of the physiological signal detection device 1 (such a simple structure embodiment being shown in Figure 7 and a combined embodiment being shown in Figure 9) in such a way that the lower flange 41 of the edge enclosure band 40 is coupled to an edge portion of a bottom surface of the base layer 20 and the upper flange 42 of the edge enclosure band 40 is coupled to an edge portion of a top surface of the electrode pad 11, in which the edge portion of the top surface of the electrode pad 11 corresponds to the edge portion of the bottom surface of the base layer 20 in order to facilitate enclosure and retention effected by the edge enclosure band 40 and to improve comfortableness and aesthetics and help attaching to the garment 50.

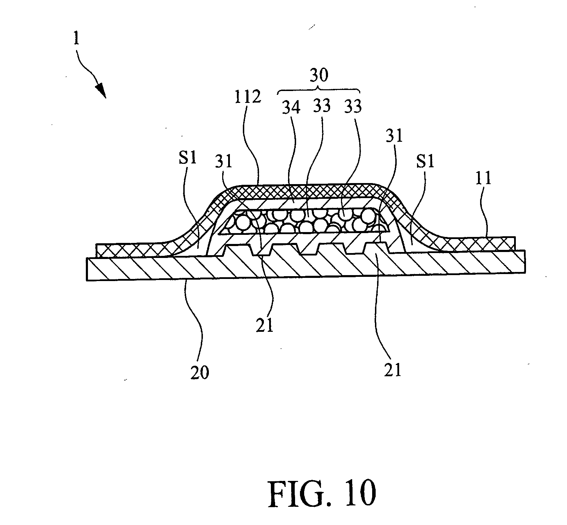

Further, as shown in Figure 10, the water absorption unit 30 may further comprises at least one water-preservation inner lining 33 and an accommodation sack 34 enclosing the water-preservation inner lining 33 in order to prevent fast loss of water. In an embodiment where a plurality of water-preservation inner linings 33 is included, to prevent a loose arrangement of the plurality of water-preservation inner linings 33, the accommodation sack 34 is used to enclose and confine the water-preservation inner linings 33 so that the water absorption unit 30 forms a structure that is adjustable and supports the electrode pad 11, this featuring both water preservation and supporting. When the present invention is attached to the human body surface, the water absorption unit 30 tightly abut against the corresponding electrode pad 11 to comply with and tightly engage with the curves of body surface, eliminating any potential gap therebetween, whereby electrical current on the body surface can be easily conducted to the electrode pad 11 to enhance the detection of variation of physiological signal.

The accommodation sack 34 has a bottom that is also provided with a plurality of spaced anti-skidding sections 31 and the top surface of the base layer 20 forms a plurality of spaced counterpart anti-skidding sections 21. The counterpart anti-skidding sections 21 respectively correspond to the anti-skidding sections 31. The counterpart anti-skidding sections 21 are respectively engageable with the anti-skidding sections 31 so as to prevent uneven raised configuration on an outside surface of the electrode pad 11 due to sliding of the water absorption unit 30.

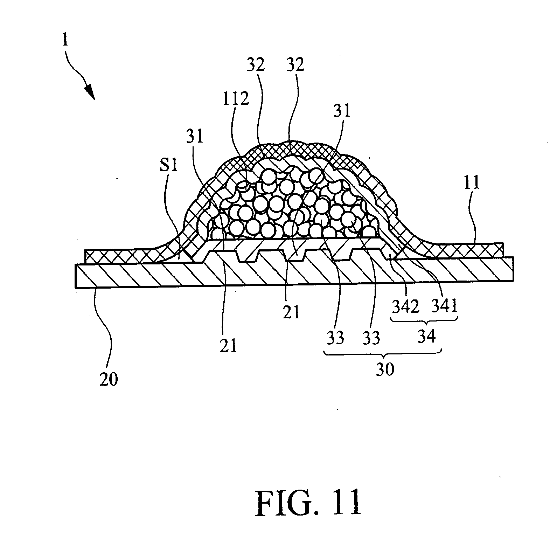

To prevent the water of the water absorption unit 30 from being fast lost and to enhance wetting of the electrode pad 11 by the water absorption unit 30, as shown in Figure 11, the accommodation sack 34 comprises an upper water-penetrating layer 341 and a lower water-proof layer 342 that overlap each other. The lower water-proof layer 342 is set in engagement with the base layer 20 and the upper water-penetrating layer 341 is in engagement with the electrode pad 11. The plurality of anti-skidding sections 31 is formed on a bottom surface of the lower water-proof layer 342. The upper water-penetrating layer 341 is a fabric layer, paper layer, or semi-permeable membrane resin layer. As such, water is only allowed to discharge through the accommodation sack 34' and the electrode pad 11 and this facilitates keeping water in the accommodation sack 34.

The water-preservation inner lining 33 is a component made of cotton paper, cotton fabric, silica gel, water-absorbing foam, fluff paste, sodium polyacrylate or other polymers of propenoic acid that show an equivalent function, or superabsorbent polymers. The shape of the component can be variable forms (such as sphere).

In the above discussion, the water absorption unit 30 has a top that is directly shaped as a projecting form (as shown in Figure 8) and after absorbing water, the water absorption unit 30 bulges and shows a more distinctly projecting form to directly support the electrode pad 11. As shown in Figure 11, if the water absorption unit 30 is made in the form comprising a plurality of water-preservation inner linings 33 in combination with an accommodation sack 34, then the water-preservation inner linings 33, after absorbing water, become bulging to make a plurality of projecting portions 32 on the surface of the accommodation sack 34. Such projecting portions 32 are also capable of supporting the corresponding electrode pad 11 and this makes it more compliant to the curves of body surface for more tight engagement.

As such, the present invention provides a physiological signal detection device, which comprises a combined structure of at least one electrode pad 11, a base layer 20, and at least one water absorption unit 30 to provide water absorbing and wet keeping functions, by which fast loss of water is prevented and wetting of the electrode pad 11 is enhanced. Further, the water absorption unit 30 is capable of bulging by absorbing water to show a projecting form, so that an effect of easy contact and tight engagement of the electrode pad with human body surface is achieved to ease the detection of physiological signal of an inspection subject and improve convenience of use. Further, an edge enclosure band 40 is also included to provide function of positioning, improving comfortableness and aesthetics, and reducing influence on conduction of electrical current and lowering noise interference.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A physiological signal detection device (1), comprising:
a base layer (20);
at least one electrode pad (11), which is positioned on a top surface of the base layer (20), the electrode pad (11) and the base layer (20) forming a first receiving compartment (S1) therebetween; and
at least one water absorption unit (30), which is positioned in the first receiving compartment (S1), the water absorption unit (30) having a top engaging the electrode pad (11), the water absorption unit (30) having a bottom engaging the base layer (20), **characterized in that** the water absorption unit (30) comprises at least one water-preservation inner lining (33) and an accommodation sack (34) enclosing the water-preservation inner lining (33) the accommodation sack (34) having a bottom forming a plurality of spaced anti-skidding sections (31), the top surface of the base layer (20) forming a plurality of spaced counterpart anti-skidding sections (21), the counterpart anti-skidding sections (21) respectively corresponding to the anti-skidding sections (31), the counterpart anti-skidding sections (21) being respectively engageable with the anti-skidding sections (31).

2. The physiological signal detection device (1) as claimed in Claim **1,** wherein the electrode pad (11) is formed by weaving a plurality of non-conductive fibrous yarns and a plurality of conductive fibrous yarns, the plurality of conductive fibrous yarns of the electrode pad (11) being woven to form a conductive zone (112).

3. The physiological signal detection device (1) as claimed in Claim **1,** wherein the electrode pad (11) is entirely made by weaving a plurality of conductive fibrous yarns.

4. The physiological signal detection device (1) as claimed in Claim **1,** wherein the water absorption unit (30) comprises one of cotton paper, cotton fabric, silica gel, and water-absorbing foam.

5. The physiological signal detection device (1) as claimed in Claim **1,** wherein the water absorption unit (30) comprises a component made of sodium polyacrylate.

6. The physiological signal detection device (1) as claimed in Claim **1,** wherein the base layer (20) comprises a water-proof layer.

7. The physiological signal detection device as claimed in Claim **1,** wherein the base layer comprises an upper water-proof layer (22) and a lower fabric layer (23) that overlap each other, the upper water-proof layer (22) being set in engagement with the water absorption unit (30).

8. The physiological signal detection device (1) as claimed in Claim **1,** wherein the at least one electrode pad (11) includes a plurality of electrode pads (11), which are arranged, in a mutually spaced manner, on the top surface of the base layer (20), each of the electrode pads (11) and the base layer (20) forming therebetween a first receiving compartment (S1), the at least one water absorption unit (30) including a plurality of water absorption units (30), each of the water absorption units (30) being positioned in each of the first receiving compartments (S1), each of the water absorption units (30) having a top engaging each of the electrode pads (11), each of the water absorption units (30) having a bottom engaging the base layer (20), a connection band being connected to two adjacent ones of the electrode pads (11), the connection band (12) and the base layer (20) forming therebetween a second receiving compartment (S2), the electrode pads (11) and the connection band (12) being connected to each other to form a top layer (10) corresponding in shape to the base layer (20).

9. The physiological signal detection device (1) as claimed in Claim **8,** wherein each of the electrode pads (11) is further provided with at least one extension conductor (13), which has an end extending into and electrically with each of the electrode pads (11) and an opposite end extending beyond one side of each of the electrode pads (11).

10. The physiological signal detection device (1) as claimed in Claim **1** further comprising an edge enclosure band (40), the edge enclosure band (40) having a lower flange (41) coupled to an edge portion of a bottom surface of the base layer (20) and an upper flange (42) coupled to an edge portion of a top surface of the electrode pad (11), the edge portion of the top surface of the electrode pad (11) corresponding to the edge portion of the bottom surface of the base layer (20).

11. The physiological signal detection device (1) as claimed in Claim **1**, wherein the water-preservation inner lining (33) comprises one of cotton paper, cotton fabric, silica gel, and water-absorbing foam.

12. The physiological signal detection device (1) as claimed in Claim **1,** wherein the water-preservation inner lining (33) comprises a component made of sodium polyacrylate.

13. The physiological signal detection device (1) as claimed in Claim **1,** wherein the accommodation sack (34) comprises an upper water-penetrating layer (341) and a lower water-proof layer (342) that overlap each other, the lower water-proof layer being (342) set in engagement with the base layer (20), the upper water-penetrating layer (341) being set in engagement with the electrode pad (11), the plurality of anti-skidding sections (31) being formed on a bottom surface of the lower water-proof layer (342).

14. The physiological signal detection device (1) as claimed in Claim **13**, wherein the upper water-penetrating layer (341) comprises one of a fabric layer, a paper layer, or a semi-permeable membrane resin layer.

## Patentansprüche

1. Eine physiologische Signaldetektionsvorrichtung (1), umfassend:
eine Basisschicht (20);
zumindest ein Elektrodenpad (11), wobei dieses auf einer oberen Oberfläche der Basisschicht (20) positioniert ist, das Elektrodenpad (11) und die Basisschicht (20) bilden einen ersten Aufnahmeraum (S1) dazwischen; und
zumindest eine Wasserabsorptionseinheit (30), die in dem ersten Aufnahmeraum (S1) positioniert ist, die Wasserabsorptionseinheit (30) hat eine Oberseite, die mit dem Elektrodenpad (11) in Verbindung steht, die Wasserabsorptionseinheit (30) hat einen Boden, der mit der Basisschicht (20) in Verbindung steht, **dadurch gekennzeichnet, dass** die Wasserabsorptionseinheit (30) zumindest eine wasserbewahrende innere Abdichtung (33) und einen Aufnahmesack (34) umfasst, der die wasserbewahrende innere Abdichtung (33) umschließt, der Aufnahmesack (34) hat einen Boden, der eine Mehrzahl von beabstandeten Antirutschbereichen (31) ausbildet, die obere Oberfläche der Basisschicht (20) bildet eine Mehrzahl von beabstandeten Gegenstück-Antirutschbereichen (21) aus, die Gegenstück-Antirutschbereiche (21) entsprechen jeweils den Antirutschbereichen (31), die Gegenstück-Antirutschbereiche (21) sind entsprechend mit den Antirutschbereichen (31) verbindbar.

2. Die physiologische Signaldetektionsvorrichtung (1) nach Anspruch 1, wobei das Elektrodenpad (11) durch Verweben einer Mehrzahl von nicht-leitenden faserförmigen Garnen und einer Mehrzahl von leitfähigen faserförmigen Garnen gebildet wird, die Mehrzahl der leitfähigen faserförmigen Garne des Elektrodenpads (11) sind verwoben, um eine leitende Zone (112) auszubilden.

3. Die physiologische Signaldetektionsvorrichtung (1) nach Anspruch 1, wobei das Elektrodenpad (11) gänzlich durch Verweben einer Mehrzahlt von leitfähigen faserförmigen Garnen hergestellt wird.

4. Die physiologische Signaldetektionsvorrichtung (1) nach Anspruch 1, wobei die Wasserabsorptionseinheit (30) jeweils ein Baumwollpapier, einen Baumwollstoff, ein Silikatgel und einen wasserabsorbierenden Schaum umfasst.

5. Die physiologische Signaldetektionseinheit (1) nach Anspruch 1, wobei die Wasserabsorptionseinheit (30) eine Komponente hergestellt aus Natriumpolyacrylat umfasst.

6. Die physiologische Signaldetektionseinheit (1) nach Anspruch 1, wobei die Basisschicht (20) eine wasserdichte Schicht umfasst.

7. Die physiologisch Signaldetektionseinheit (1) nach Anspruch 1, wobei die Basisschicht eine obere wasserdichte Schicht (22) und eine untere Stoffschicht (23) umfasst, die sich überlappen, wobei die obere wasserdichte Schicht (22) in Verbindung mit der Wasserabsorptionseinheit (30) steht.

8. Die physiologische Signaldetektionseinheit (1) nach Anspruch 1, wobei das zumindest eine Elektrodenpad (11) eine Mehrzahl von Elektrodenpads (11) umfasst, die in einer wechselseitig beabstandeten Art und Weise auf der oberen Oberfläche einer Basisschicht (20) angeordnet sind, jedes der Elektrodenpads (11) und der Basisschicht (20) bilden dazwischen einen ersten Aufnahmeraum (S1), die zumindest eine Wasserabsorptionseinheit (30) umfasst eine Mehrzahl von Wasserabsorptionseinheiten (30), jede der Wasserabsorptionseinheiten (30) ist in jedem der ersten Aufnahmeräume (S1) positioniert, jede der Wasserabsorptionseinheiten (30) hat eine Oberseite, die mit dem jeweiligen Elektrodenpad (11) in Verbindung steht, jede der Wasserabsorptionseinheiten (30) hat einen Boden, der mit der Basisschicht (20) in Verbindung steht, ein Verbindungsband ist mit zwei benachbarten Elektrodenpads (11) verbunden, das Verbindungsband (12) und die Basisschicht (20) bilden dazwischen einen zweiten Aufnahmeraum (S2), die Elektrodenpads (11) und das Verbindungsband (12) sind miteinander verbunden, um eine obere Schicht (10) auszubilden, die in der Form der Basisschicht (20) entspricht.

9. Die physiologische Signaldetektionsvorrichtung (1) nach Anspruch 8, wobei jedes der Elektrodenpads (11) des Weiteren zumindest mit einem Erweiterungsleiter (13) versehen ist, der ein Ende hat, das sich in das jeweilige Elektrodenpad (11) erstreckt und damit elektrisch verbunden ist, und ein gegenüberliegendes Ende, das sich über eine Seite des jeweiligen Elektrodenpads (11) hinaus erstreckt.

10. Die physiologische Signaldetektionsvorrichtung (1) nach Anspruch 1, weiter umfassend ein Randabdeckungsband (40), das Randabdeckungsband (40) hat einen unteren Flansch (41), der mit einem Randbereich einer Bodenoberfläche der Basisschicht (20) gekoppelt ist, und einen oberen Flansch (42), der mit einem Randbereich der oberen Oberfläche des Elektronenpads (11) gekoppelt ist, der Randbereich der oberen Oberfläche des Elektrodenpads (11) entspricht dem Randbereich der Bodenoberfläche der Basisschicht (20).

11. Die physiologische Signaldetektionsvorrichtung (1) nach Anspruch 1, wobei die wasserbewahrende innere Abdeckung (33) ein Baumwollpapier, einen Baumwollstoff, ein Silikatgel und einen wasserabsorbierenden Schaum umfasst.

12. Die physiologische Signaldetektionsvorrichtung (1) nach Anspruch 1, wobei die wasserbewahrende innere Abdichtung (33) eine Komponente hergestellt aus Natriumpolyacrylat umfasst.

13. Die physiologische Signaldetektionsvorrichtung (1) nach Anspruch 1, wobei der Aufnahmesack (34) eine obere wassereindringende Schicht (341) und eine untere wasserdichte Schicht (342) umfasst, die einander überlappen, die untere wasserdichte Schicht (342) steht in Verbindung mit der Basisschicht (20), die obere wassereindringende Schicht (341) steht in Verbindung mit dem Elektrodenpad (11), die Mehrzahl der Antirutschbereiche (31) ist an einer Bodenoberfläche der unteren wasserdichten Schicht (342) ausgebildet.

14. Die physiologische Signaldetektionsvorrichtung (1) nach Anspruch 13, wobei die obere wassereindringende Schicht (341) aus einer Stoffschicht, einer Papierschicht oder einer semipermeablen Membran-Harzschicht besteht.

## Revendications

1. Dispositif de détection de signal physiologique (1), comprenant :
une couche de base (20) ;
au moins une électrode (11), qui est positionnée sur une surface supérieure de la couche de base (20), l'électrode (11) et la couche de base (20) formant un premier compartiment de réception (S1) entre celles-ci ; et
au moins une unité à absorption d'eau (30), qui est positionnée dans le premier compartiment de réception (S1), l'unité à absorption d'eau (30) comportant une partie supérieure entrant en prise avec l'électrode (11), l'unité à absorption d'eau (30) comportant une partie inférieure entrant en prise avec la couche de base (20), **caractérisé en ce que** l'unité à absorption d'eau (30) comprend au moins un revêtement intérieur pour la préservation d'eau (33) et un sac de logement (34) enfermant le revêtement intérieur pour la préservation d'eau (33), le sac de logement (34) comportant une partie inférieure formant une pluralité de sections anti-glissement espacées (31), la surface supérieure de la couche de base (20) formant une pluralité de sections anti-glissement homologues espacées (21), les sections anti-glissement homologues (21) correspondant respectivement aux sections anti-glissement (31), les sections anti-glissement homologues (21) pouvant respectivement entrer en prise avec les sections anti-glissement (31).

2. Dispositif de détection de signal physiologique (1) selon la revendication 1, dans lequel l'électrode (11) est formée en tissant une pluralité de fils fibreux non conducteurs et une pluralité de fils fibreux conducteurs, la pluralité de fils fibreux conducteurs de l'électrode (11) étant tissés pour former une zone conductrice (112).

3. Dispositif de détection de signal physiologique (1) selon la revendication 1, dans lequel l'électrode (11) est entièrement faite en tissant une pluralité de fils fibreux conducteurs.

4. Dispositif de détection de signal physiologique (1) selon la revendication 1, dans lequel l'unité à absorption d'eau (30) comprend un parmi du papier de coton, du tissu de coton, du gel de silice, et de la mousse absorbante d'eau.

5. Dispositif de détection de signal physiologique (1) selon la revendication 1, dans lequel l'unité à absorption d'eau (30) comprend un composant fait de polyacrylate de sodium.

6. Dispositif de détection de signal physiologique (1) selon la revendication 1, dans lequel la couche de base (20) comprend une couche étanche à l'eau.

7. Dispositif de détection de signal physiologique selon la revendication 1, dans lequel la couche de base comprend une couche supérieure étanche à l'eau (22) et une couche de tissu inférieure (23), qui se chevauchent, la couche supérieure étanche à l'eau (22) étant placée en prise avec l'unité à absorption d'eau (30).

8. Dispositif de détection de signal physiologique (1) selon la revendication 1, dans lequel l'au moins une électrode (11) comprend une pluralité d'électrodes (11), qui sont agencées, de manière mutuellement espacée, sur la surface supérieure de la couche de base (20), chacune des électrodes (11) et la couche de base (20) formant entre celles-ci un premier compartiment de réception (S1), l'au moins une unité à absorption d'eau (30) comprenant une pluralité d'unités à absorption d'eau (30), chacune des unités à absorption d'eau (30) étant positionnée dans chacun des premiers compartiments de réception (S1), chacune des unités à absorption d'eau (30) comportant une partie supérieure entrant en prise avec chacune des électrodes (11), chacune des unités à absorption d'eau (30) comportant une partie inférieure entrant en prise avec la couche de base (20), une bande de raccordement étant raccordée à deux électrodes (11) adjacentes, la bande de raccordement (12) et la couche de base (20) formant entre celles-ci un second compartiment de réception (S2), les électrodes (11) et la bande de raccordement (12) étant raccordées les unes aux autres pour former une couche haute (10) dont la forme correspond à celle la couche de base (20).

9. Dispositif de détection de signal physiologique (1) selon la revendication 8, dans lequel chacune des électrodes (11) est en outre pourvue d'au moins un conducteur à prolongement (13), dont une extrémité s'étend dans et électriquement avec chacune des électrodes (11) et une extrémité opposée s'étend au-delà d'un côté de chacune des électrodes (11).

10. Dispositif de détection de signal physiologique (1) selon la revendication 1 comprenant en outre une bande d'enceinte de bord (40), la bande d'enceinte de bord (40) comportant un épaulement inférieur (41) accouplé à une partie à bord d'une surface inférieure de la couche de base (20) et un épaulement supérieur (42) accouplé à une partie à bord d'une surface supérieure de l'électrode (11), la partie à bord de la surface supérieure de l'électrode (11) correspondant à la partie à bord de la surface inférieure de la couche de base (20).

11. Dispositif de détection de signal physiologique (1) selon la revendication 1, dans lequel le revêtement intérieur pour la préservation d'eau (33) comprend un parmi du papier de coton, du tissu de coton, du gel de silice, et de la mousse à absorption d'eau.

12. Dispositif de détection de signal physiologique (1) selon la revendication 1, dans lequel le revêtement intérieur pour la préservation d'eau (33) comprend un composant fait de polyacrylate de sodium.

13. Dispositif de détection de signal physiologique (1) selon la revendication 1, dans lequel le sac de logement (34) comprend une couche supérieure à pénétration d'eau (341) et une couche inférieure étanche à l'eau (342) qui se chevauchent, la couche inférieure étanche à l'eau (342) étant placée en prise avec la couche de base (20), la couche supérieure à pénétration d'eau (341) étant placée en prise avec l'électrode (11), la pluralité de sections anti-glissement (31) étant formées sur une surface inférieure de la couche inférieure étanche à l'eau (342).

14. Dispositif de détection de signal physiologique (1) selon la revendication 13, dans lequel la couche supérieure à pénétration d'eau (341) comprend une parmi une couche de tissu, une couche de papier, ou une couche de résine de membrane semi-perméable.
